# FASCICULE DE BREVET EUROPEEN

(11) **EP 1 846 570 B1**
(45) Date de publication et mention de la délivrance du brevet: **01.06.2011**
(21) Numéro de dépôt: 06709294.0
(22) Date de dépôt: 10.02.2006
(51) Int. Cl.: C12Q 1/34

(54) **PROCEDE DE DETECTION ET/OU DE MESURE A HAUT DEBIT D'UNE ACTIVITE LIPASIQUE OU PHOSPHOLIPASIQUE**
VERFAHREN ZUM NACHWEIS UND/ODER ZUR MESSUNG EINER LIPASE- ODER PHOSPHOLIPASEAKTIVITÄT MIT HOHER GESCHWINDIGKEIT
METHOD FOR THE HIGH-SPEED DETECTION AND/OR MEASUREMENT OF A LIPASE OR PHOSPHOLIPASE ACTIVITY

(30) Priorité: 11.02.2005 FR 0501425
(43) Date de publication de la demande: 24.10.2007
(73) Titulaire: CENTRE NATIONAL DE LA RECHERCHE SCIENTIFIQUE, 75794 Paris Cedex 16 (FR)
(72) Inventeur: VERGER, Robert, F-13009 Marseille (FR); SERVEAU-AVESQUE, Carole, F-13013 Marseille (FR); CHAHINIAN, Henri, F-13005 Marseille (FR)
(74) Mandataire: Grosset-Fournier, Chantal Catherine
(86) Numéro de dépôt international: PCT/FR2006/000310
(87) Numéro de publication internationale: WO 2006/085009

(56) Documents cités:
- EP-A- 0 245 799
- US-A1- 2004 014 133
- PENCREAC'H G ET AL: "An ultraviolet spectrophotometric assay for measuring lipase activity using long-chain triacyglycerols from Aleurites fordii seeds" ANALYTICAL BIOCHEMISTRY, ACADEMIC PRESS, NEW YORK, NY, US, vol. 303, no. 1, 2002, pages 17-24, XP002990175 ISSN: 0003-2697 cité dans la demande
- BEISSON FREDERIC ET AL: "Methods for lipase detection and assay: A critical review" EUROPEAN JOURNAL OF LIPID SCIENCE AND TECHNOLOGY, vol. 102, no. 2, février 2000 (2000-02), pages 133-153, XP002354344 ISSN: 1438-7697
- TSUZUKI TSUYOSHI ET AL: "alpha-Eleostearic acid (9Z11E13E-18:3) is quickly converted to conjugated linoleic acid (9Z11E-18:2) in rats" JOURNAL OF NUTRITION, vol. 134, no. 10, October 2004 (2004-10), pages 2634-2639, ISSN: 0022-3166

## Description

La présente invention a pour objet un procédé de détection et/ou de mesure *in vitro* à haut débit d'une activité lipasique ou phospholipasique, ainsi que ses applications.

L'élaboration de méthodes analytiques pour la détection et le dosage des lipases a fait l'objet de travaux depuis de nombreuses années (Beisson F., Tiss A., Rivière C. and Verger R. (2000) Methods for lipase detection and assay : a critical review. Eur. J. Lipid Sci.Technol. 2: 133-153).

Des triglycérides naturellement fluorescents pour détecter de très faibles activités lipasiques ont été utilisés dans le but de caractériser les propriétés cinétiques et énantiosélectives des lipases générées par la technique du "phage display" (Beisson F., Ferté N., Nari J., Noat G., Arondel V. and Verger R. (1999). Use of naturally fluorescent triacylglycerols from Parinari glaberrimum to detect low lipase activities from Arabidopsis thaliana seedlings. J. Lipid Res. 40: 2313-2321).

Le principe de cette méthode repose d'une part sur la présence dans l'huile de parinarium d'un acide gras naturellement fluorescent (acide parinarique) qui possède quatre doubles liaisons conjuguées et par conséquent absorbe la lumière dans le domaine ultraviolet et la réémet par un phénomène de fluorescence. Sous l'action hydrolytique des lipases, l'acide parinarique est libéré du triglycéride d'origine pour être solubilisé dans une phase micellaire. Ce changement de phase (d'une phase émulsifiée vers une phase micellaire) s'accompagne d'une variation d'émission spectrale de fluorescence qui a été mise à profit pour suivre, avec une grande sensibilité, l'évolution au cours du temps de la réaction d'hydrolyse.

Une des limitations de cette méthode fluorescente est liée à l'oxydabilité par l'oxygène atmosphérique des quatre doubles liaisons conjuguées de l'acide parinarique. C'est pour cela que les Inventeurs ont transposé le même principe de mesure à une huile extraite du bois de Chine (Tung oil) et utilisée depuis l'antiquité dans la fabrication des laques chinoises. Cette huile contient une très grande proportion d'acide α-éléostéarique qui lui possède seulement trois doubles liaisons conjuguées et n'est pas fluorescent mais absorbe la lumière ultraviolette. Cet acide est également beaucoup moins oxydable que l'acide parinarique. C'est ainsi que les Inventeurs ont utilisé d'une part les propriétés d'absorption ultraviolette de l'acide alpha-éléostéarique et d'autre part, le changement de phase décrit précédemment (d'une phase émulsifiée vers une phase micellaire) pour mettre au point une méthode dite en « émulsion » décrite dans l'article de Pencreac'h G., Graille J., Pina M. and Verger R. (2002) An ultraviolet spectrophotometric assay for measuring lipase activity using long-chain triacylglycerols from Aleurites fordii seeds. Anal. Biochem, 303 : 17-24.

US20040014133 décrit des procédés de détection d'une activité lipasique par p.e. SPA ou fluorescence et obtient une bonne reproducibilité en liant le substrat après modification sur la phase solide (exemple biotine).

Dans le cadre du développement de ces travaux, les Inventeurs ont utilisé les triglycérides purifiés de l'huile de tung pour tapisser d'un film très mince (équivalent à quelques centaines de couches monomoléculaires) les puits de plaques de microtitration (constitués d'une matière plastique non absorbante dans l'ultraviolet). Les Inventeurs ont démontré que ce film mince de triglycérides reste parfaitement adsorbé sur les parois des puits, même après rinçage par différents tampons aqueux. Sous l'action hydrolytique (à l'interface huile-eau) de différentes lipases, comme précédemment décrit, l'acide α-éléostéarique est libéré et solubilisé en phase micellaire. Par conséquent, son spectre d'absorption ultraviolet est modifié et d'autre part, le chemin optique est considérablement augmenté par suite du passage de l'état adsorbé à l'état soluble, ce qui constitue un avantage significatif pour la technique par revêtement ou « coating ».

La présente invention résulte de la mise en évidence par les Inventeurs du fait que cette nouvelle technique par revêtement ou « coating » de lipides (triglycérides naturels ou esters de synthèse) présente par rapport à la technique en « émulsion » précédente, les avantages suivants :
- une meilleure conservation au cours du temps des substrats lipidiques adsorbés (ou « coatés ») dans les puits des plaques de microtitration,
- une activité des lipases plus élevée sur le substrat « coaté » que sur le substrat en émulsion,
- une meilleure reproductibilité des expériences avec le substrat « coaté »,
- un rapport signal/bruit plus favorable dû à l'augmentation du chemin optique par suite du passage de l'état adsorbé à l'état soluble.

Ainsi, la présente invention a pour but de fournir une nouvelle méthode de mesure de l'activité lipasique qui est plus spécifique, quantitative et sensible, que les méthodes décrites jusqu'à ce jour dans ce domaine, et qui permet de mesurer de très faibles activités lipolytiques (équivalentes à une quantité minimale d'environ 2 ng pour la Lipase de *Thermomyces lanuginosus,* « TLL »), en utilisant un substrat naturel des lipases.

La présente invention a également pour but de fournir une nouvelle méthode de mesure de l'activité lipasique permettant de cribler à haut débit les nombreux mutants des lipases susceptibles d'être générés grâce à la technique du "phage display".

L'invention a aussi pour but de fournir une nouvelle méthode de mesure de l'activité lipasique à haut débit pour sélectionner des mutants et des lipases énantiosélectives, à l'aide des esters chiraux, d'intérêt pharmaceutique et biotechnologique, contenant de l'acide alpha-éléostéarique. Ces mutants ainsi sélectionnés pourront servir de catalyseurs enzymatiques pour la synthèse asymétrique de molécules d'intérêt pharmaceutique et agro-alimentaire.

L'invention a également pour but de fournir une nouvelle méthode de mesure de l'activité lipasique utilisable en clinique humaine, pour la mesure de la lipasémie plasmatique, lors du diagnostic précoce de diverses affections pancréatiques.

L'invention concerne un procédé de détection et/ou de mesure *in vitro* d'une activité lipasique ou phospholipasique caractéristique d'une lipase ou phospholipase d'origine naturelle ou synthétique dans un échantillon susceptible de contenir cette lipase ou phospholipase, caractérisé en ce qu'il comprend :
- l'addition de l'échantillon susmentionné susceptible de contenir ladite lipase ou phospholipase en solution aqueuse, dans les puits de plaques de microtitration revêtus d'une couche d'environ 0,5 à environ 5 µm, et de préférence d'environ 1 µm d'épaisseur d'un substrat lipidique pouvant être hydrolysé par ladite lipase ou phospholipase en libérant de l'acide α-éléostéarique qui se solubilise en phase micellaire dans ladite solution aqueuse,
- la détection et/ou la mesure de l'activité lipasique ou phospholipasique par spectrophotométrie dans le spectre d'absorption UV de l'acide α-éléostéarique libéré lors de l'étape précédente.

Par lipase ou phospholipase d'origine naturelle ou synthétique on entend toute lipase ou phospholipase de mammifères ou de microorganismes (bactéries, champignons...), le cas échéant modifiée par mutation d'un ou plusieurs acides aminés. L'activité lipasique peut être classiquement mesurée selon les méthodes décrites notamment dans Beisson et coll. (Beisson F., Tiss A., Rivière C. and Verger R. (2000) Methods for lipase detection and assay : a critical review. Eur. J. Lipid Sci. Technol. 2: 133-153). L'activité lipasique ou phospholipasique peut être par exemple mesurée par la méthode décrite dans Wolf et coll. (Wolf C., Sagaert L. et Bereziat G. (1981) A sensitive assay of phospholipases using the fluorescent probe 2-parinaroyllecithin. Biochem. Biophys. Res. Com. 99: 275-283) où un phospholipide de synthèse contenant de l'acide parinarique a été utilisé pour mesure l'activité enzymatique d'une phospholipase A2 de venin de serpent.

L'invention concerne plus particulièrement un procédé tel que défini ci-dessus, caractérisé en ce que le substrat lipidique pouvant être hydrolysé par ladite lipase ou phospholipase en libérant de l'acide α-éléostéarique, est un lipide choisi parmi des molécules d'intérêt industriel et/ou pharmaceutique sur lequel sont liés de façon covalente des groupes α-éléostéarates, et est notamment choisi parmi :
- les triglycérides purifiés de l'huile de tung de formule générale 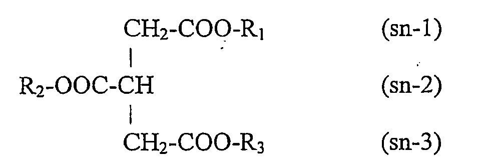 où R₁ et R₃ représentent des résidus d'acide gras identiques ou différents, comprenant d'environ 12 à 20 atomes de carbone, et de préférence 18 atomes de carbone, et présentant éventuellement une ou plusieurs insaturations, et où R₂ représente l'acide α-éléostéarique.
- les di- et monoglycérides, ou des esters de cholestérol ou des phospholipides contenant 1 ou 2 chaînes d'α-éléostéarate en position adéquate selon le type de régiosélectivité des lipases ou des phospholipases recherchées,
- l'α-éléostéarate de citronellol de formule suivante : 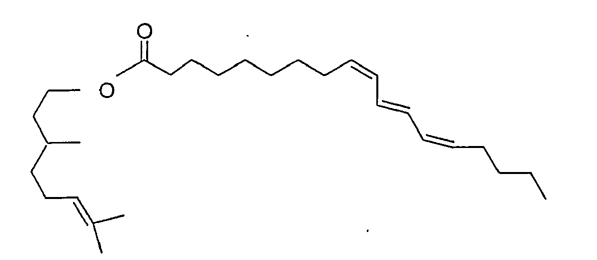
- ou des esters d'acide α-éléostéarate avec d'autres alcools ou molécules prochirales ou chirales d'intérêt pharmaceutique comme le propanolol, le sotalol ou le carvedilol utilisés comme β-bloquant, ou avec des molécules d'intérêt industriel comme le menthol (dérivé terpénique d'intérêt aromatique).

En particulier, les triglycérides de synthèse où R₁ et R₃ tels que définis ci-dessus représentent des résidus d'acides gras identiques ou différents, comprenant environ 12 à 20 atomes de carbone, et de préférence 18 atomes de carbone, et présentant éventuellement une ou plusieurs insaturations, et où R₂ représente l'acide α-eléostéarique permettent de cribler des lipases sn-2 spécifiques (c'est-à-dire capable de libérer l'acide gras présent en position sn-2 des triglycérides). De façon comparable, les phospholipides de synthèse contenant de l'acide α-éléostéarique en position sn-1 et/ou sn-2 permettent de mesurer les activités enzymatiques des phospholipases A1 et/ou des phospholipases A2, une phospholipase A1 étant définie comme une enzyme capable de libérer l'acide gras présent en position sn-1 des glycérophospholipides et une phospholipase A2 étant définie comme une enzyme capable de libérer l'acide gras présent en position sn-2 des glycérophospholipides.

Dans l'huile de tung, les acides gras en quantité importante sont : l'acide α-éléostéarique (70-80%), l'acide oléique (10 %) et l'acide linoléique (15 %), comme décrit notamment dans Radunz et coll. (A. Radunz, P. He and G. H. Schmid, Analysis of the seed lipids of Aleurites montana. Z. Naturforsch 53 (1998), pp. 305-310).

L'invention concerne également un procédé tel que défini ci-dessus, caractérisé en ce qu'il comprend, préalablement à l'addition de l'échantillon susceptible de contenir la lipase ou phospholipase dans les puits de plaques de microtitration, une étape d'ajout aux puits des plaques de microtitration revêtus du substrat lipidique, d'une solution tampon constituée de Tris et de sels biliaires (NaTDC) et, le cas échéant de β-cyclodextrine, notamment dans les proportions suivantes : NaTDC (4 mM) et β-Cyclodextrine (3 mg/mL).

L'invention a également pour objet un procédé tel que défini ci-dessus, caractérisé en ce que les plaques de microtitration revêtues du substrat lipidique sont telles qu'obtenues par :
- addition d'une composition comprenant le substrat lipidique en solution dans un solvant approprié susceptible de pouvoir être évaporé sous vide, tel que l'hexane ou l'éther de pétrole, cette addition étant le cas échéant effectuée après lavage des puits desdites plaques avec ledit solvant,
- évaporation sous vide dudit solvant jusqu'à la formation d'un revêtement dudit substrat lipidique de 0,5 à 5 µm, et de préférence de 1 µm d'épaisseur sur les parois des puits des plaques de microtitration.

L'invention a plus particulièrement pour objet un procédé tel que défini ci-dessus, de mesure *in vitro* de la lipasémie plasmatique chez l'homme ou l'animal, caractérisé en ce que l'échantillon contenant la lipase est un échantillon sanguin prélevé sur l'homme ou l'animal.

La présente demande décrit également l'application d'un procédé tel que défini ci-dessus, au diagnostic *in vitro* de pathologies liées à une augmentation du taux plasmatique de lipase chez l'homme ou l'animal, par rapport aux taux plasmatiques de cette lipase chez un individu sain.

L'invention concerne plus particulièrement l'application susmentionné d'un procédé tel que défini ci-dessus, au diagnostic *in vitro:*
- d'affections pancréatiques telles que la pancréatite aiguë, pancréatite chronique, caractérisées par une augmentation du taux plasmatique de lipase chez l'homme ou l'animal, par rapport aux taux plasmatiques dé cette lipase chez un individu sain,
- ou de l'insuffisance rénale, les traumatismes de l'abdomen (ischémie, infarctus mésentérique, perforation ou occlusion intestinale).

L'invention concerne également un procédé de préparation de plaques de microtitration comprenant des puits revêtus d'un substrat lipidique pouvant être hydrolysé par une lipase ou phospholipase en libérant de l'acide α-éléostéarique, caractérisé en ce qu'il comprend les étapes suivantes :
- addition aux puits desdites plaques d'une composition comprenant le substrat lipidique en solution dans un solvant approprié susceptible de pouvoir être évaporé sous vide, tel que l'hexane ou l'éther de pétrole, cette addition étant le cas échéant effectuée après lavage des puits desdites plaques avec ledit solvant,
- évaporation sous vide dudit solvant jusqu'à la formation d'un revêtement dudit substrat lipidique d'environ 0,5 à environ 5 µm, et de préférence d'environ 1 µm d'épaisseur sur les parois des puits des plaques de microtitration.

L'invention a également pour objet les plaques de microtitration comprenant des puits revêtus d'un substrat lipidique pouvant être hydrolysé par une lipase ou phospholipase en libérant de l'acide α-éléostéarique, telles qu'obtenues par le procédé susmentionné, le revêtement dudit substrat lipidique sur les parois des puits des plaques de microtitration étant de 0,5 à 5 µm, et de préférence de 1 µm d'épaisseur.

L'invention a plus particulièrement pour objet l'utilisation des plaques de microtitration susmentionnées comprenant des puits revêtus d'un substrat lipidique, pour la mise en oeuvre d'un procédé de détection et/ou de mesure *in vitro* d'une activité lipasique ou phospholipasique tel que défini ci-dessus, ou pour la mise en oeuvre d'un procédé de criblage d'inhibiteurs d'enzymes lipolytiques (inhibiteurs de lipases ou de phospholipases) comprenant une étape de mesure de leur éventuelle capacité à inhiber lesdites enzymes grâce au procédé de détection et/ou de mesure *in vitro* d'une activité lipasique ou phospholipasique selon l'invention.

### LÉGENDE DES FIGURES

La Figure 1 représente les protocoles de préparation des microplaques : "Coating" *versus* émulsion. Le substrat lipidique est adsorbé dans chaque puits de la microplaque ("Coating") ou préparé en émulsion puis déposé dans la microplaque (Emulsion).

Les Figures 2a et 2b représentent la stabilité du substrat lipidique adsorbé ou émulsifié.

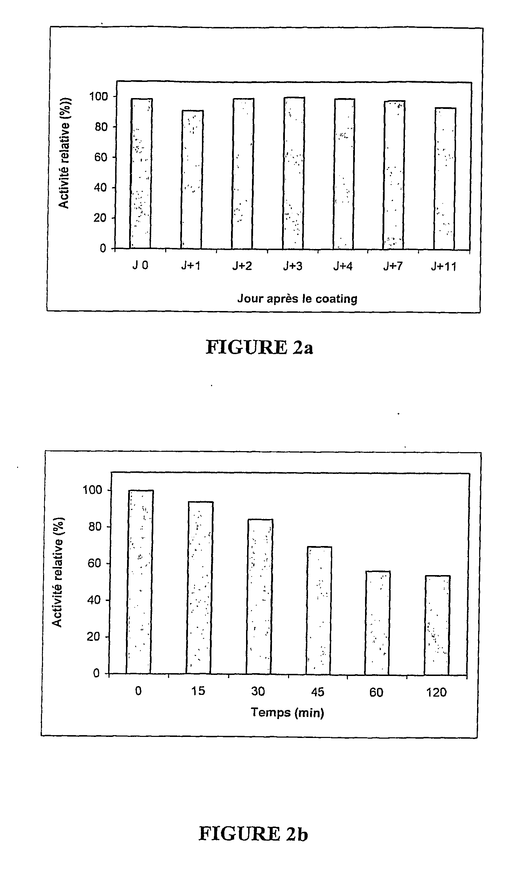

Dans la Figure 2a, la microplaque a été préparée par "coating" du S citronellol-α-éléostéarate ester (80 µg/ puits) et conservée plusieurs jours à 4°C. La stabilité du coating a été étudiée par dosage de l'activité lipolytique de la lipase de *Thermomyces lanuginosus* (TLL) (10 nM finale) à différents jours après le "coating" de l'ester dans un tampon Tris 10 mM pH 8.0, CaCl₂ 6 mM, EDTA 1 mM, BHT 0.001% et β-cyclodextrine 3 mg/mL. La Figure 2a représente l'activité enzymatique relative (%) pendant plusieurs jours après le "coating".

Dans la Figure 2b, des émulsions à 20 µg/mL d'α-éléostéarate de S citronellol ont été réalisées et distribuées (200 µL) dans chaque puits de la plaque de microtitration. La stabilité des émulsions est testée en mesurant l'activité de la TLL à différents temps après l'émulsification, comme décrit ci-dessus pour la Figure 2a.

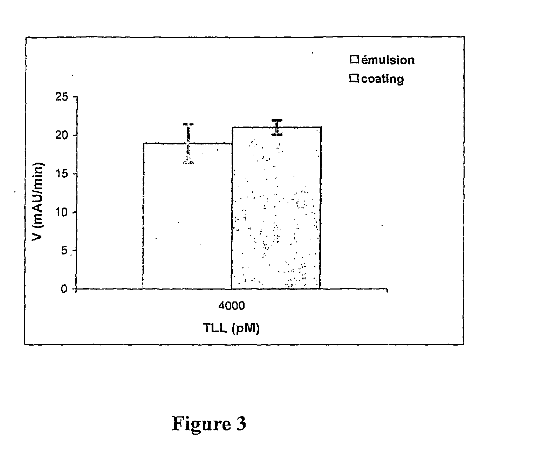

La Figure 3 représente la reproductibilité des activités de la TLL dosées sur le S citronellol-α-éléostéarate ester « coaté » ou en émulsion. L'activité de la TLL (4 nM) a été testée sur le S citronellol-α-éléostéarate ester adsorbé (80 µg/puits) ou en émulsion (20 µg/mL) en microplaque dans un tampon Tris 10 mM pH 8.0, CaCl₂ 6 mM, EDTA 1 mM, BHT 0.001 % et β-cyclodextrine 3 mg/mL. Les vitesses sont exprimées en milli-unités d'absorbance apparues par minute (mAU/min). La variabilité des résultats a été calculée sur 8 expériences pour chacune des conditions expérimentales.

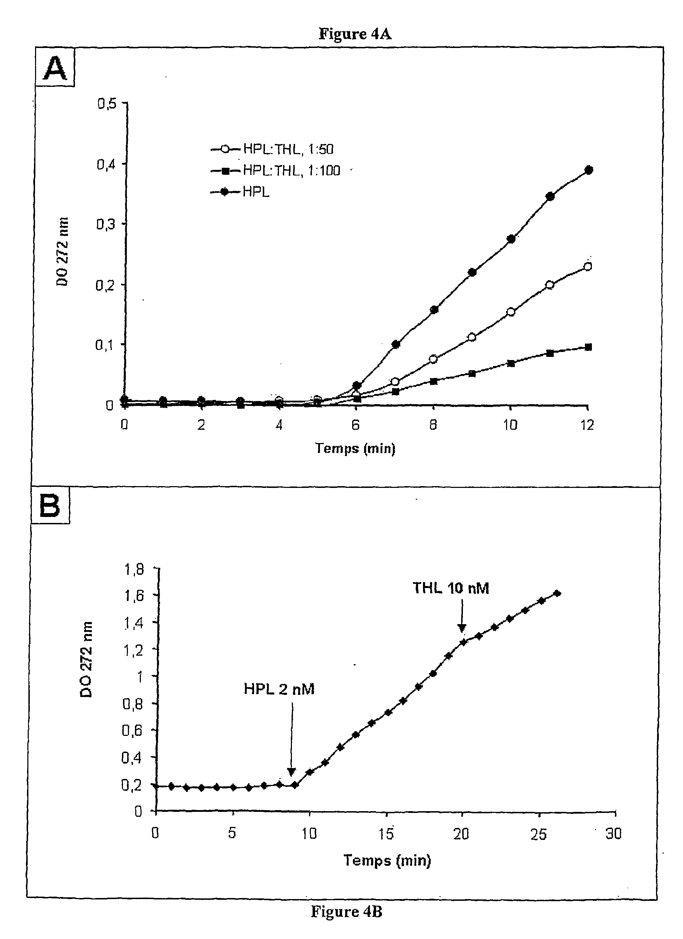

Les Figures 4A et 4B représentent la cinétique d'inhibition de la HPL (lipase pancréatique humaine) par la THL (tétrahydrolipstatine). La microplaque est préparée par « coating » des triglycérides (50 µg/puits) extraits à partir de l'huile de tung qui contient une forte proportion d'acide α-éléostéarique. Une solution (200 µl) de tampon Tris est ajoutée dans les puits. L'activité de la HPL seule, ou pré-incubée avec la THL, est mesurée par l'enregistrement de la densité optique (méthode A ; figure 4A). La Figure 4A représente la densité optique (DO) à 272 nm en fonction du temps. La courbe avec les ronds noirs correspond à la lipase HPL seule ; la courbe avec les carrés noirs correspond à la lipase HPL pré-incubée avec la THL dans un rapport de 1:100 et la courbe avec les ronds blancs correspond à la lipase HPL pré-incubée avec la THL dans un rapport de 1:50.

La Figure 4B correspond à un protocole différent (méthode B) : après injection de la HPL (à la concentration finale de 2 nM), la THL est injectée dans le milieu réactionnel (à la concentration finale de 10 nM) en cours de lipolyse.

L'invention sera davantage illustrée à l'aide de la description détaillée qui suit de la mise en oeuvre d'une méthode de mesure d'une activité lipase à l'aide des plaques de microtitration selon l'invention (méthode dite de « coating »), par comparaison avec une méthode de mesure dite par « émulsion ».

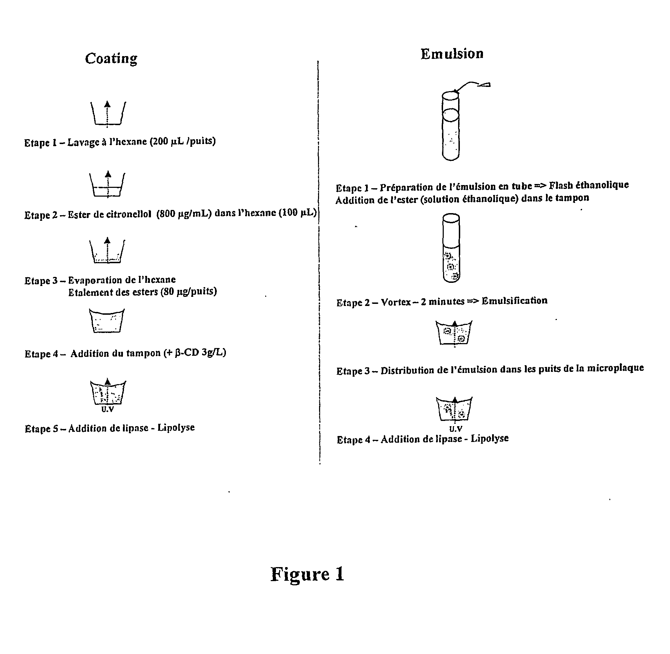

Cette étude a été réalisée en menant en parallèle l'étude de l'activité lipasique soit par « coating » de lipides (triglycérides naturels ou esters de synthèse) soit sur des émulsions réalisées au préalable dans un tube, avant répartition d'échantillons dans les puits de la microplaque (Figure 1).

L'étude comparée « coating » *versus* « émulsion » en utilisant des esters énantiomériques (α-éléostéarate de R ou S citronellol) montre clairement que le « coating » de ces substrats sur la microplaque présente plusieurs avantages :

### 1. Meilleure conservation au cours du temps des substrats coatés

Une solution d'α-éléostéarate de citronellol a été « coatée » (80 µg/puits) dans les 96 puits d'une microplaque. L'activité de la lipase de *Thermomyces lanuginosus* (TLL) (10 nM, concentration finale) a ensuite été testée à différents jours après le « coating ». La microplaque a été conservée à 4°C, avec ou sans tampon dans les puits. Sur le graphe de la figure 2a, on peut remarquer que l'activité de la lipase demeure constante au cours du temps, indiquant que la fixation du substrat sur la microplaque est reproductible et stable au moins pendant 20 jours.

En parallèle, des émulsions à 20 µg/mL d'α-éléostéarate de S citronellol sont réalisées en injectant 12 µL d'une solution d'ester (5 mg/mL en éthanol-BHT (Butylated HydroxyToluene) 0,001%) dans le tampon d'activité de la lipase (qsp 3 mL), créant ainsi un flash éthanolique. La solution est ensuite agitée avec un « vortex» pendant 2 minutes. La stabilité des émulsions est testée en mesurant l'activité de la TLL (10 nM) à différents temps après l'émulsification. L'activité de la lipase n'est pas constante au cours du temps et elle diminue significativement 30 minutes après la fabrication de l'émulsion (Figure 2b). Ces résultats indiquent que les émulsions ne peuvent pas être conservées et donc doivent être utilisées immédiatement après leur fabrication. Ceci est un inconvénient majeur par rapport au « coating » des substrats en microplaque qui peut être réalisé plusieurs jours à l'avance.

### 2. Activité des lipases plus élevée sur le substrat « coaté » que sur l'émulsion

L'activité de la lipase est plus élevée avec le substrat « coaté » qu'avec le substrat en émulsion (voir Figure 3).

### 3. Meilleure reproductibilité des expériences avec le substrat « coaté »

La dispersion des résultats obtenue par le calcul des moyennes et des écarts-types sur 8 expériences est plus importante avec les substrats en émulsion (Figure 3). De plus, la fabrication des émulsions est difficile à obtenir de manière reproductible (cf. ci-dessus).

En conclusion, le développement de ce test en microplaque est une méthode représentant un caractère innovant par rapport à toutes les méthodes précédentes (Beisson et coll., Eur. J. Lipid Sci. Technol. 2000, 2 : 133 - 153) pour le dosage rapide et continu des lipases avec des triglycérides naturels, reposant sur les propriétés spectrales dans l'UV de l'acide α-éléostéarique. C'est un test sensible et reproductible. Le « coating » du substrat permet de préparer à l'avance une microplaque et de la conserver au moins deux semaines en chambre froide, sans précautions particulières. Par contre la technique d'émulsification, telle que publiée par Pencreac'h et collaborateurs (Anal Biochem. 2002; 303: 17-24), présente de nombreux inconvénients, à savoir instabilité des émulsions, activité lipolytique plus faible....

Par rapport à la publication précédente (Pencreac'h et coll., Anal Biochem. 2002; 303: 17-24), la présente invention propose deux améliorations techniques importantes :
- Haut débit : Adaptation du principe de la méthode publiée (Pencreac'h et coll., Anal Biochem. 2002; 303: 17-24) dans un système à haut débit (microplaque 96 puits).
- « Coating » de substrats lipidiques dans les puits des plaques de microtitration avec une durée de conservation considérablement plus grande.

### UTILISATION DES PLAQUES DE L'INVENTION POUR LA MISE EN OEUVRE D'UN PROCEDE DE CRIBLAGE D'INHIBITEURS D'ENZYMES LIPOLYTIQUES

Les plaques de microtitration comprenant des puits revêtus d'un substrat lipidique pouvant être hydrolysé par une lipase ou une phospholipase A1 ou A2 en libérant de l'acide α-éléostéarique, obtenues selon le procédé de l'invention, sont utilisées pour le criblage à haut débit d'inhibiteurs et la mesure de l'inhibition des lipases ou des phospholipases A1 ou A2.

### Exemple de la tétrahydrolipstatine (Orlistat ou THL) : inhibiteur puissant des lipases digestives

(voir les articles suivants : Digestive lipases inhibition: an in vitro study. Tiss A., Miled N., Verger R., Gargouri Y., Abousalham A., 2004, Lipases and phospholipases in drug development from biochemistry to molecular pharmacology, Wiley VCH (Muller G. and Petry S., Eds), 155, 193 ; Covalent inactivation of lipases. Ransac S., Gargouri Y., Marguet F., Buono G., Beglinger C., Hildebrand P., Lengsfeld H., Hadvary P., Verger R., 1997, Methods in Enzymol., 286, 190-231).

Les plaques de microtitration, revêtues (« coating ») du substrat lipidique, sont préparées comme cela a été décrit ci-dessus. Une solution constituée de tampon Tris et de β-cyclodextrine (3 mg/ml) est ajoutée dans les puits. La densité optique à 270 nm est enregistrée en fonction du temps pendant 5 à 10 min (voir figures 4A et 4B). Une solution de lipase pancréatique humaine (HPL), seule ou pré-incubée avec de la THL (inhibiteur), est injectée dans les puits. L'activité enzymatique est alors enregistrée en fonction du temps par la mesure de la DO à 272 nm (méthode A ; figure 4A). La pré-incubation de la HPL avec la THL, à un excès molaire de 1 pour 100 ou de 1 pour 50 pendant 10 min, réduit l'activité lipasique initiale de 75% ou 40% respectivement (voir figure 4A).

L'effet de la THL sur l'activité lipasique a aussi été mesuré en injectant la THL au cours de la lipolyse (méthode B ; figure 4B). L'injection de la THL (à la concentration finale de 10 nM) réduit l'activité de la HPL d'environ 40%.

### CONCLUSION

Au vu des résultats présentés dans les figures 4A et 4B, on constate que la présente invention est parfaitement adaptée à la mesure à haut débit de l'inhibition des lipases, et donc au criblage d'inhibiteurs de lipases et de phospholipasess A1 et A2.

## Revendications

1. Procédé de préparation de plaques de microtitration comprenant des puits revêtus d'un substrat lipidique, pouvant être hydrolysé par une lipase ou phospholipase en libérant de l'acide α-éléostéarique, **caractérisé en ce qu'**il comprend les étapes suivantes :
- addition aux puits desdites plaques d'une composition comprenant le substrat lipidique en solution dans un solvant approprié susceptible de pouvoir être évaporé sous vide, cette addition étant le cas échéant effectuée après lavage des puits desdites plaques avec ledit solvant,
- évaporation sous vide dudit solvant jusqu'à la formation d'un revêtement dudit substrat lipidique de 0,5 à 5 µm, et de préférence 1 µm d'épaisseur sur les parois des puits des plaques de microtitration.

2. Plaques de microtitration comprenant des puits revêtus d'un substrat lipidique pouvant être hydrolysé par une lipase ou phospholipase en libérant de l'acide α-éléostéarique, obtenues par le procédé selon la revendication 1, le revêtement dudit substrat lipidique sur les parois des puits des plaques de microtitration étant 0,5 à 5 µm, et de préférence 1 µm d'épaisseur.

3. Procédé de détection et/ou de mesure *in vitro* d'une activité lipasique ou phospholipasique caractéristique d'une lipase ou phospholipase d'origine naturelle ou synthétique dans un échantillon susceptible de contenir cette lipase ou phospholipase, **caractérisé en ce qu'**il comprend :
- l'addition d'une solution tampon constituée de Tris et de sels biliaires (NaTDC) et, le cas échéant, de β-cyclodextrine, dans les puits de plaques de microtitration obtenues selon la revendication 1,
- l'addition de l'échantillon susmentionné susceptible de contenir ladite lipase ou phospholipase en solution aqueuse, dans les puits de plaques de microtitration obtenues selon la revendication 1, revêtus d'une couche d'un substrat lipidique pouvant être hydrolysé par ladite lipase ou phospholipase en libérant de l'acide α-éléostéarique qui se solubilise en phase micellaire dans ladite solution aqueuse,
- la détection et/ou la mesure de l'activité lipasique ou phospholipasique par spectrophotométrie dans le spectre d'absorption UV de l'acide α-éléostéarique libéré lors de l'étape précédente.

4. Procédé selon la revendication 3, **caractérisé en que** le substrat lipidique pouvant être hydrolysé par ladite lipase ou phospholipase en libérant de l'acide α-éléostéarique, est choisi parmi :
- les triglycérides purifiés de l'huile de tung de formule générale 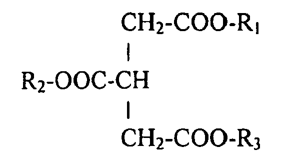 où R1 et R3 représentent des résidus d'acide gras identiques ou différents, comprenant environ 12 à 20 atomes de carbone, et de préférence 18 atomes de carbone, et présentant éventuellement une ou plusieurs insaturations, et où R2 représente l'acide α-éléostéarique,
- les di- et monoglycérides, ou des esters de cholestérol ou des phospholipides contenant 1 ou 2 chaînes d'α-éléostéarate en position adéquate selon le type de régiosélectivité des lipases ou des phospholipases recherchées,
- l'α-éléostéarate de citronellol de formule suivante : 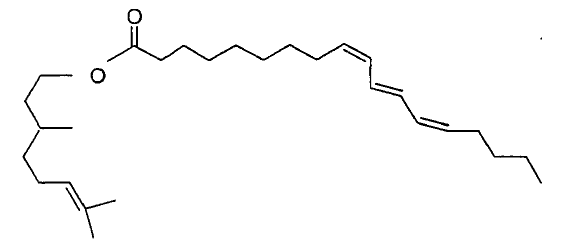
- ou des esters d'acide α-éléostéarate avec d'autres alcools ou molécules prochirales ou chirales d'intérêt pharmaceutique comme le propanolol, le sotalol ou le carvedilol, ou avec des molécules d'intérêt industriel comme le menthol.

5. Procédé selon la revendication 3 ou 4, de mesure *in vitro* de la lipasémie plasmatique chez l'homme ou l'animal, **caractérisé en ce que** l'échantillon contenant la lipase est un échantillon sanguin prélevé sur l'homme ou l'animal.

6. Application d'un procédé selon l'une des revendications 3 à 5, au diagnostic *in vitro :*
- d'affections pancréatiques telles que la pancréatite aiguë, la pancréatite chronique, **caractérisées par** une augmentation du taux plasmatique de lipase chez l'homme ou l'animal, par rapport aux taux plasmatiques de cette lipase chez un individu sain,
- ou de l'insuffisance rénale, les traumatismes de l'abdomen (ischémie, infarctus mésentérique, perforation ou occlusion intestinale).

7. Utilisation des plaques selon la revendication 2, pour la mise en oeuvre d'un procédé de détection et/ou de mesure *in vitro* d'une activité lipasique ou phospholipasique tel que défini dans l'une des revendications 3 à 5, ou pour la mise en oeuvre d'un procédé de criblage d'inhibiteurs d'enzymes lipolytiques.

## Claims

1. Method of preparation of microtitration plates comprising wells coated with a lipid substrate which is able to be hydrolyzed by a lipase or phospholipase in releasing alpha-eleostearic acid, **characterized in that** it comprises the following steps:
- the addition to the wells of said plates of a composition comprising the lipid substrate in solution in an appropriate solvent capable of being evaporated off under vacuum, this addition being if appropriate carried out after washing the wells of said plates with said solvent,
- evaporation under vacuum of said solvent until the formation of a coating of said lipid substrate of 0.5 to 5 µm, and preferably 1 µm in thickness on the walls of the wells of the microtitration plates.

2. Microtitration plates comprising wells coated with a lipid substrate which is able to be hydrolyzed by a lipase or phospholipase in releasing α-eleostearic acid, obtained by the method of claim 1, the coating of said lipid substrate on the walls of the wells of the microtitration plates being 0.5 to 5 µm, and preferably 1 µm in thickness.

3. Method for the *in vitro* detection and/or measurement of a lipase or phospholipase activity characteristic of a natural or synthetic origin lipase or phospholipase in a sample likely to contain said lipase or phospholipase, **characterized in that** it comprises:
- the addition of a buffer solution constituted by Tris and bile salts (NaTDC) and, if appropriate, β-cyclodextrin, in the wells of microtitration plates, obtained according to claim 1,
- the addition of the above-mentioned sample likely to contain said lipase or phospholipase in aqueous solution, into the wells of microtitration plates obtained according to claim 1, coated with a layer of a lipid substrate which is able to be hydrolyzed by said lipase or phospholipase in releasing α-eleostearic acid which is solved in the micellar phase in said aqueous solution,
- the detection and/or the measurement of the lipase or phospholipase activity by spectrophotometry in the UV absorption spectrum of the α-eleostearic acid released during the previous step.

4. Method according to claim 3, **characterised in that** the lipid substrate which is able to be hydrolyzed by said lipase or phospholipase in releasing α-eleostearic acid, is chosen from:
- the purified triglycerides of tung oil of general formula 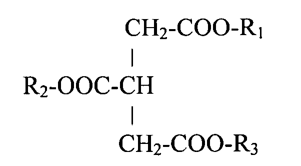 where R₁ and R₃ represent identical or different fatty acids residues, comprising approximately 12 to 20 carbon atoms, and preferably comprising 18 carbon atoms, and optionally having one or more unsaturations, and where R₂ represents α-eleostearic acid,
- the di- and monoglycerides, or the cholesterol esters or phospholipids containing 1 or 2 α-eleostearate chains in a suitable position according to the type of regioselectivity of the sought lipases or phospholipases,
- the citronellol α-eleostearate of the following formula: 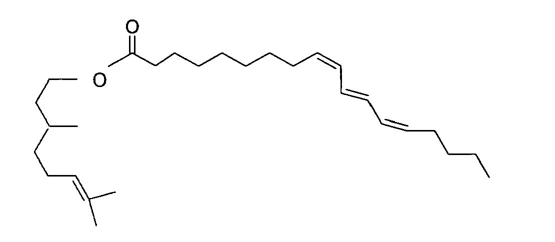
- or the acid esters of α-eleostearate with other alcohols or pharmaceutical interested prochiral or chiral molecules such as propanolol, sotalol or carvedilol, or with industrial interested molecules such as menthol.

5. Method according to claims 3 or 4, of *in vitro* measurement of the plasma lipasemia in humans or animals, **characterized in that** the sample containing the lipase is a blood sample taken from a human or an animal.

6. Application of a method according to one of claims 3 to 5, to *in vitro* diagnostic:
- of pancreatic diseases such as acute pancreatitis, chronic pancreatitis, **characterized by** an increase of plasma lipase level in humans or animals, compared to plasma lipase level in a healthy individual,
- or of renal failure, abdominal trauma (ischemia, mesenteric infarct, intestinal perforation or occlusion).

7. Use of plates according to claim 2, for the implementation of a method for the *in vitro* detection and/or measurement of a lipase or phospholipase activity as defined in one of claims 3 to 5, or for the implementation of a method for screening inhibitors of lipolytic enzymes.

## Patentansprüche

1. Verfahren zum Herstellen von Mikrotiterplatten, die Vertiefungen umfassen, die mit einem Lipidsubstrat beschichtet sind, das durch eine Lipase oder Phospholipase hydrolysiert werden kann, indem α-Eläostearinsäure freigesetzt wird, **dadurch gekennzeichnet, dass** es die folgenden Schritte umfasst:
- Zugeben zu den Vertiefungen einer Zusammensetzung, die das Lipidsubstrat in Lösung in einem geeigneten Lösemittel umfasst, das in der Lage ist, unter Vakuum verdampft zu werden, wobei dieses Zugeben gegebenenfalls nach dem Waschen der Vertiefungen der Platten mit dem Lösemittel durchgeführt wird,
- Verdampfen des Lösemittels unter Vakuum bis zur Bildung einer Beschichtung des Lipidsubstrats mit einer Dicke von 0,5 bis 5 µm, und bevorzugt 1 µm, auf den Wänden der Vertiefungen der Mikrotiterplatten.

2. Mikrotiterplatten, die Vertiefungen umfassen, die mit einem Lipidsubstrat beschichtet sind, das mit einer Lipase oder Phospholipase hydrolysiert werden kann, indem α-Eläostearinsäure freigesetzt wird, die durch das Verfahren gemäß Anspruch 1 erhalten wurden, wobei die Lipidsubstratbeschichtung auf den Wänden der Vertiefungen der Mikrotiterplatten eine Dicke von 0,5 bis 5 µm und bevorzugt 1 µm aufweist.

3. in-vitro-Verfahren zum Nachweisen und/oder Messen einer Lipase- oder Phospholipaseaktivität, das durch eine Lipase oder Phospholipase natürlichen oder synthetischen Ursprungs in einer Probe **gekennzeichnet** ist, die in der Lage ist, diese Lipase oder Phospholipase zu enthalten, **dadurch gekennzeichnet, dass** es Folgendes umfasst:
- Zugeben einer Pufferlösung, die aus Tris und Gallensalzen (NaTDC) und gegebenenfalls β-Cyclodextrin besteht, in die Vertiefungen der Mikrotiterplatten, die nach Anspruch 1 erhalten wurden,
- Zugeben der oben genannten Probe, die in der Lage ist, die Lipase oder Phospholipase in wässriger Lösung zu enthalten, in die Vertiefungen der Mikrotiterplatten, die nach Anspruch 1 erhalten wurden, die mit einer Schicht eines Lipidsubstrats beschichtet sind, das durch die Lipase oder Phospholipase hydrolysiert werden kann, indem α-Eläostearinsäure freigesetzt wird, die sich in mizellarer Phase in der wässrigen Lösung solubilisiert,
- Nachweisen und/oder Messen der Lipase- oder Phospholipaseaktivität durch Spektrophotometrie im UV-Absorptionsspektrum der während des vorhergehenden Schritts freigesetzten α-Eläostearinsäure.

4. Verfahren nach Anspruch 3, **dadurch gekennzeichnet, dass** das Lipidsubstrat, das durch die Lipase oder Phospholipase hydrolysiert werden kann, indem α-Eläostearinsäure freigesetzt wird, ausgewählt ist aus:
- gereinigten Tungöltriglyceriden mit der allgemeinen Formel: 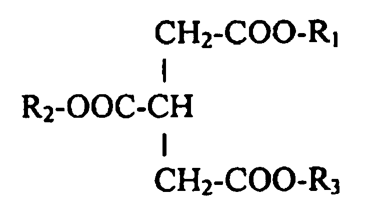 worin R₁ und R₃ für gleiche oder verschiedene Fettsäurereste stehen, die etwa 12 bis 20 Kohlenstoffatome und bevorzugt 18 Kohlenstoffatome umfassen, und gegebenenfalls eine oder mehrere Ungesättigtheiten aufweisen und worin R₂ für α-Eläostearinsäure steht,
- den Cholesteroldi- und -monoglyceriden oder - estern oder Pospholipiden, die 1 oder 2 α-Eläostearinketten in der entsprechender Position gemäß dem Regioselektivitätstyp der gesuchten Lipasen oder Phospholipasen enthalten,
- dem α-Eläostearin von Citronellol mit der folgenden Formel: 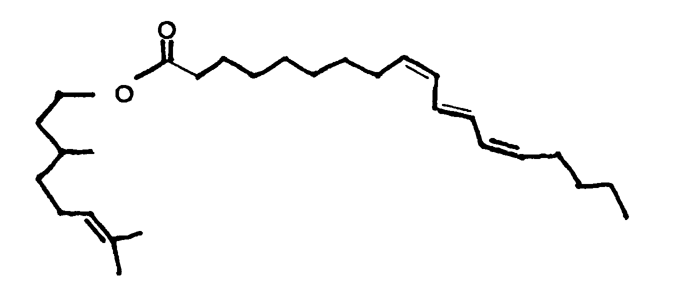
- oder den Estern von α-Eläostearinsäure mit anderen Alkoholen oder prochiralen oder chiralen Molekülen von pharmazeutischem Interesse wie Propanolol, Sotalol oder Carvedilol, oder mit Molekülen von industriellem Interesse, wie Menthol.

5. Verfahren nach Anspruch 3 oder 4 zum In-vitro-Messen der plasmatischen Lipasämie beim Menschen oder beim Tier, **dadurch gekennzeichnet, dass** die Probe, die die Lipase enthält, eine Blutprobe ist, die dem Menschen oder dem Tier entnommen wurde.

6. Anwenden eines Verfahrens nach einem der Ansprüche 3 bis 5 zur *In-vitro-Diagnose:*
- von Bauchspeicheldrüsenerkrankungen, wie etwa akuter Bauchspeicheldrüsenentzündung, chronischer Bauchspeicheldrüsenentzündung, die durch eine Erhöhung des Plasmalipasespiegel beim Menschen oder beim Tier, bezogen auf die Plasmaspiegel dieser Lipase bei einem gesunden Individuum **gekennzeichnet** sind,
- oder von Nierenversagen, Abdominaltraumata (Ischämie, Mesenterialinfarkt, Darmperforation oder Darmverschluss).

7. Verwenden von Platten nach Anspruch 2 zum Durchführen eines Verfahrens zum Nachweisen und/oder Messen einer Lipase- oder Phospholipaseaktivität *in vitro,* wie in einem der Ansprüche 3 bis 5 definiert, oder zum Durchführen eines Verfahrens zum Screenen von Inhibitoren fettspaltender Enzyme.
